# EUROPÄISCHE PATENTSCHRIFT

(11) **EP 2 562 237 B1**
(45) Veröffentlichungstag und Bekanntmachung des Hinweises auf die Patenterteilung: **22.08.2018**
(21) Anmeldenummer: 12004369.0
(22) Anmeldetag: 08.06.2012
(51) Int. Cl.: C10L 3/06, C10L 3/08, B01D 53/14

(54) **VERFAHREN ZUR BEREITSTELLUNG EINES GASES MIT SEHR HOHEM METHANGEHALT UND DAZU AUSGELEGTE ANLAGE**
METHOD FOR PROVIDING A GAS WITH VERY HIGH METHANE CONTENT AND ASSEMBLY FOR SAME
PROCÉDÉ DE PRÉPARATION D'UN GAZ À TRÈS FORTE TENEUR EN MÉTHANE ET INSTALLATION CONÇUE DANS CE BUT

(30) Priorität: 07.06.2011 DE 102011103430
(43) Veröffentlichungstag der Anmeldung: 27.02.2013
(73) Patentinhaber: Hitachi Zosen Inova Etogas GmbH, 70565 Stuttgart (DE)
(72) Erfinder: Buxbaum, Martin, 6861 Alberschwende (AT); Waldstein, Gregor, 70565 Stuttgart (DE)
(74) Vertreter: Leinweber & Zimmermann

(56) Entgegenhaltungen:
- WO-A1-2011/060539
- WO-A1-2012/003849
- GB-A- 1 389 981
- US-A1- 2010 286 292

## Beschreibung

Die Erfindung betrifft ein Verfahren zur Bereitstellung eines Gases mit sehr hohem Methangehalt mittels einer an einem ersten Aufbereitungsort erfolgenden Abtrennung wenigstens eines Teils des Kohlendioxids aus einem sowohl Kohlendioxid als auch Methan enthaltenden Gasstrom sowie eine zur Durchführung des Verfahrens geeignete Anlage und ein mit Hilfe dieses Verfahrens hergestelltes Gasgemisch. Derartige Verfahren sind allgemein gut bekannt, wobei einzelnen technischen Ausgestaltungen solcher Verfahren unterschiedliche Prinzipien zugrundeliegen können, wie die Druckadsorption oder eine Aminwäsche.

Ein bekanntes Anwendungsgebiet eines solchen Verfahrens liegt in der Gasaufbereitung eines von einer Biogasanlage hergestellten Rohbiogases, bei der dieses zu Biomethan mit einem sehr hohen Methangehalt aufbereitet wird. Ein typisches Rohbiogas enthält ca. zwischen 45 und 70% Methan, während nach Aufbereitung ein Methangehalt bis zu 98% und höher erreichbar ist. Die Prozentangaben beziehen sich in dieser Anmeldung immer auf Volumenprozent. Im übrigen wird in dieser Anmeldung vereinfacht von Gasen und Gasströmen auch dann gesprochen, wenn die Gase keine reinen Gase, sondern Gasgemische aus unterschiedlichen Komponenten sind.

Das so durch die Abtrennung des Kohlendioxids gewonnene Gasgemisch mit sehr hohem Methananteil, das beim Ausgangsstoff Rohbiogas hierin auch als Biomethan bezeichnet wird, kann gespeichert werden und dazu insbesondere in ein vorhandenes Gasnetz eingespeist werden, nachdem es zuvor auf die erforderlichen Einspeisebedingungen konditioniert wurde, denen das Gasnetz unterliegt. Ein Beispiel für eine solche Gastrennung und nachfolgende Einspeisung ist in der DE 2009 018126 A1 offenbart.

Das abgetrennte Kohlendioxid kann entweder in die Atmosphäre entlassen werden, oder auch als Treibhausgas an insbesondere unterirdische Speicherstätten gepumpt werden. In der DE 10 2009 018126 A1 wird darüber hinaus vorgeschlagen, das abgetrennte Kohlendioxid zur Bildung des Kohlenstoffanteils eines Eduktgases zu verwenden, welches nach Wasserstoffzufuhr katalytisch methanisiert werden kann und als Produktgas ebenfalls in ein Gasnetz einspeisbar ist.

GB 1 389 981 A offenbart ein Verfahren nach dem Oberbegriff von Anspruch 1.

Der Erfindung liegt die Aufgabe zugrunde, das eingangs genannte Gasbereitstellungsverfahren insbesondere unter dem Gesichtspunkt der Qualität des bereitgestellten Gases zu verbessern.

In verfahrenstechnischer Hinsicht wird diese Aufgabe durch eine Weiterbildung des Verfahrens der eingangs genannten Art mit den Merkmalen von Anspruch 1 gelöst. Es ist vorgesehen, dass ein an einem zweiten Aufbereitungsort durch katalytische Methanisierung eines sowohl Kohlendioxid als auch Wasserstoff enthaltenden Eduktgases erzeugtes Produktgas wenigstens einen Teil des Gasstromes vor dessen Erreichen des ersten Aufbereitungsortes bildet.

Auf diese Weise kann, obwohl das am zweiten Aufbereitungsort durch katalytische Methanisierung entstandene Produktgas bereits durchaus hohe Methangehalte von erreichbaren 92% aufweist, ein nochmals höherer Methangehalt im Endprodukt nach der Kohlendioxidabtrennung erreicht werden und dadurch umgekehrt auch eine geringere Restsumme der üblichen weiteren Bestandteile des Produktgases nach Methanisierung, insbesondere von Kohlendioxid und Wasserstoff, erreicht werden. Bei der Methanisierung des Eduktgases finden im wesentlichen die folgenden, in Summe CH₄ bildenden Reaktionen statt,
1) CO + H₂O ↔ CO₂ + H₂, die sogenannte water-gas shift reaction
2) CO + 3H₂ ↔ CH₄ + H₂O, die CO-Methanisierung und
3) CO₂ + 4H₂ ↔ CH₄ + 2H₂O, die CO₂-Methanisierung,
deren Gleichgewichtsbedingung den maximalen CH₄-Gehalt des Produktgases limitiert.

Erfindungsgemäß leitet man aus dem Gasstrom abgetrenntes Kohlendioxid dem zweiten Aufbereitungsort zu, wo dieses wenigstens einen Teil des Kohlendioxids des Eduktgases bildet, wobei insbesondere der gesamte Kohlendioxidanteil des Eduktgases aus diesem abgetrennten Kohlendioxid gebildet sein kann. So kann das aus dem Abgasstrom abgetrennte Kohlendioxid als Quelle zur weiteren Methanherstellung genutzt werden, und die Methanherstellung erfolgt kohlendioxidneutral und damit klimafreundlich.

In einer zweckmäßigen Ausgestaltung wird auch Wasserstoff zumindest teilweise aus dem Gasstrom abgetrennt und insbesondere dem Eduktgas zugeführt. Dadurch wird zum einen Wasserstoff eingespart, zum anderen wird durch die doppelte Rückführung von Kohlendioxid wie auch Wasserstoff höheren Qualitätsanforderungen an das Produktgas hinsichtlich dessen Kohlendioxid- und Wasserstoffgehalts genügt.

Erfindungsgemäß führt man zur Bildung des Gasstromes das Eduktgas einem bereits Kohlendioxid und Methan enthaltenden Basisgas zu. Somit kann das katalytische Methanisierungsverfahren am zweiten Aufbereitungsort die ohnehin für die Aufbereitung des Basisgases am ersten Aufbereitungsort vorhandene, für das Abtrennverfahren ausgelegte Trenneinrichtung mitnutzen. Insbesondere lässt sich eine Methanisierung am zweiten Aufbereitungsort als Ergänzung einer bereits am ersten Aufbereitungsort bestehenden Trenneinrichtung erweitern. Bevorzugt wird dabei das Mengenverhältnis aus eingekoppeltem Produktgas zu Basisgas auf 1:1 oder kleiner, bevorzugt 1:2 oder kleiner, insbesondere 1:3 oder kleiner gesetzt, um unerwünschte Produktgasbestandteile, die nicht am ersten Aufbereitungsort oder anderweitig abgetrennt werden, geeignet zu "verdünnen". Zweckmäßig ist das Mengenverhältnis aber 1:6 oder größer, bevorzugt 1:5 oder größer, insbesondere 1:4 oder größer, um eine möglichst hohe CO₂ Wiedergewinnung in einfacher Weise zu realisieren.

In diesem Zusammenhang kann das Basisgas ein aus einer Biogasanlage gewonnenes Gas, insbesondere bereits entschwefeltes Rohbiogas sein. Derartige Anlagen haben insoweit, als eine Aufwertung des Rohbiogases zu Biomethan zur späteren Gaseinspeisung beabsichtigt ist, bereits eine Einrichtung zum Ausscheiden des in der Größenordnung von durchschnittlich 40% im Rohbiogas enthaltenen Kohlendioxids.

Das Basisgas ist jedoch nicht auf ein Rohbiogas eingeschränkt, vielmehr kommen auch andere Gasgemische in Frage, welche neben Kohlendioxid und Methan weitere Bestandteile möglichst nur in sehr geringen Konzentrationen aufweisen. Beispielsweise könnte das Basisgas auch aus einem Gasnetz entnommen werden, z.B. einem L-Gasnetz, dessen gespeichertes und transportiertes Gas aufgrund der niedrigeren geforderten Methangehaltsschwelle noch einen abtrennfähigen Kohlendioxidanteil enthält.

Grundsätzlich könnte das nach der Abtrennung am ersten Aufbereitungsort bereitgestellte Gas mit sehr hohem Methangehalt unmittelbar in der Nähe des ersten Aufbereitungsortes verwendet werden, z.B. in geeignete Behälter eingefüllt werden oder ggf. nach Zwischenspeicherung direkt zur Wandlung Gas nach Strom verwendet werden. Nach einer besonders bevorzugten Ausführungsform ist jedoch auch an eine fern dem ersten Ausführungsort liegende Nutzung des bereitgestellten Gases gedacht. Dazu wird das bereitgestellte Gas mit dem sehr hohen Methangehalt bevorzugt gemäß vorgegebenen Einspeisebedingungen für eine Einspeisung in ein den Einspeisebedingungen (gegenwärtig z.B. den Normen DVGW G 260/262/685) unterliegendes Gasnetz konditioniert und insbesondere daraufhin in das Gasnetz eingespeist.

Dabei ist eine Einspeisung als begrenzt zumischbares Zusatzgas grundsätzlich denkbar, aufgrund der erreichten sehr hohen Methangehalte wird allerdings eine Einspeisung als Austauschgas bevorzugt, das in beliebigen Mengen in das Gasnetz eingespeist werden kann, sofern eine vorherige Druckanpassung erfolgt, wie auch die Anpassung gemäß der weiteren Konditionierung hinsichtlich der Brennwertanpassung durch Zugabe eines LPG-Gases anderer Gaskennwerte (WOBBE-Index), ggf. Luftzugabe und ggf. auch eine Odorierung des Gases. Bevorzugt handelt es sich bei dem Gasnetz um ein der H-Gruppe zugeordnetes Gasnetz.

Hinsichtlich der Zusammensetzung des bereitgestellten Gases (d.h., des bereitgestellten Gasgemisches) ist ein Methangehalt von 96 vol% oder mehr, insbesondere 98 vol% oder mehr vorgesehen. Je nach Zusammensetzung des Produktgases wird insbesondere auch daran gedacht, die Trennqualität am ersten Aufbereitungsort soweit zu erhöhen, dass das bereitgestellte Gas 99 vol% oder mehr Methangehalt aufweist. Der Kohlendioxidgehalt des bereitgestellten Gases sollte dagegen 4 vol% oder weniger, bevorzugt 2 vol% oder weniger, insbesondere 1 vol% oder weniger betragen. Vergleichbare bevorzugte Grenzwerte gelten auch für den Wasserstoffgehalt des bereitgestellten Gases, wobei hier ein sehr geringer Wasserstoffgehalt von 1 vol% oder kleiner besonders bevorzugt wird. Aufgrund dieser sehr niedrigen Gehalte an Kohlendioxid und Wasserstoff genügt eine geringere Zugabe von Butan oder Propan zur Brennwerteinstellung, wodurch wiederum Kosten gespart werden können. Die obigen Maximalwerte werden prinzipiell auch für das Produktgas gewählt.

Hinsichtlich der am zweiten Aufbereitungsort erfolgenden katalytischen Methanisierung ist zweckmäßigerweise vorgesehen, dass wenigstens ein Großteil des Wasserstoffes des Eduktgases elektrolytisch erzeugt wird, insbesondere nahe dem zweiten Aufbereitungsort. Insbesondere ist daran gedacht, den gesamten Wasserstoff des Eduktgases (abgesehen von einem Wasserstoffanteil eines zum zweiten Aufbereitungsort rückgeführten Gasgemisches unmittelbar vor Ort durch Elektrolyse zu erzeugen.

Die katalytische Methanisierung am zweiten Aufbereitungsort kann in zeitlich konstantem Betrieb ablaufen, es ist aber insbesondere auch vorgesehen, die entsprechenden Methanisierungsreaktoren und Elektrolyseure für einen intermittierenden Betrieb auszulegen. Dann kann die von den Elektrolyseuren benötigte elektrische Leistung insbesondere dann abgerufen werden, wenn im Stromnetz aufgrund eines Überangebots von Strom aus regenerativen Energien (Wind oder Sonne), deren bereitgestellte elektrische Leistung deutlich schwanken kann, im Überfluss vorhanden ist. Es ist daher zweckmäßig, eine Steuerung der Zugabe des kohlendioxidhaltigen Teils des Eduktgases in Abhängigkeit der für die Elektrolyse insbesondere zeitlich schwankend zur Verfügung gestellten elektrischen Energie zu steuern. Bei Energiemangel im Netz kann CO₂ in die Atmosphäre abgelassen werden.

In einer besonders bevorzugten Ausführungsform steuert man die Methanisierung auf eine Verschiebung des Wasserstoffgehalts des Produktgases hin zu geringen Wasserstoffgehalten, möglichst 4% oder weniger, bevorzugt 3% oder weniger, insbesondere 2% oder weniger. Dies wird beispielsweise durch eine Überhöhung des Kohlendioxidgehalts des Eduktgases bezüglich der exakten Stöchiometrie der Methanherstellung aus Kohlendioxid und Wasserstoff erreicht. Dabei wird ein höherer Kohlendioxidgehalt im Produktgas in Kauf genommen, welcher durch die erfindungsgemäße Zuleitung des Produktgases vom zweiten zum ersten Aufbereitungsort und die dortige (teilweise) Abtrennung wieder ausgeglichen werden kann.

Hinsichtlich dieses am ersten Aufbereitungsort stattfindenden Abtrennung des Kohlendioxidanteils aus dem Gasstrom, oder auch zum Abtrennen des Wasserstoffes sind grundsätzlich sämtliche dazu geeigneten und aus dem Stand der Technik bekannten Verfahren einsetzbar, wie etwa Druckwasserwäsche, Druckwechseladsorption, Aminwäsche usw.. Als für die Erfindung bevorzugt angesehen wird, dass das Abtrennverfahren ohne Zugabe von Luft bei der Trennung arbeitet, da Luftzugabe (= O₂, N₂) zum CO₂-Strom dann zu Luft im Produktgas führt und die Gasqualität absenkt. Sofern ein Grundgas aus einer Biogasanlage herangezogen wird, wird eine Vorab-Entschwefelung noch in der Biogasanlage bevorzugt, so dass das am ersten Aufbereitungsort abgetrennte Kohlendioxid in möglichst reiner Form dem zweiten Aufbereitungsort zugeführt werden kann. Insbesondere können sämtliche der Rohbiogasreinigung zuzuordnenden Schritte vorab durchgeführt werden.

In vorrichtungstechnischer Hinsicht wird von der Erfindung eine Anlage zur Bereitstellung eines Gases mit sehr hohem Methangehalt vorgeschlagen, welches mit einer Einrichtung zum Abtrennen wenigstens eines Teils des Kohlendioxids aus einem der Abtrenneinrichtung zugeführten sowohl Kohlendioxid als auch Methan enthaltenden Gasstroms, einer Einrichtung zur Herstellung eines Produktgases durch katalytische Methanisierung eines sowohl Kohlendioxid als auch Wasserstoff enthaltenden Eduktgases und ein Leitungssystem aufweist, das stromaufwärts der Abtrenneinrichtung eine wenigstens teilweise Bildung des Gasstroms durch das Produktgas der Methanisierungseinrichtung erlaubt, **sowie mit den weiteren Merkmalen von Anspruch 12.**

Die Vorteile der erfindungsgemäßen Vorrichtung ergeben sich aus der obigen Beschreibung zu dem erfindungsgemäßen Verfahren, so ist die Anlage mit einer Steuereinrichtung zur Steuerung der Anlage nach einem dieser Verfahrensgestaltungen ausgestattet, je nach deren Auswahl das Leitungssystem noch eine oder mehrere der folgenden Verbindungen aufweisen kann: Eine Verbindung des Ausgangs der Abtrenneinrichtung für den abgetrennten Wasserstoff zu einem Gaseingang der Methanisierungseinrichtung; eine Verbindung eines Gasausgangs einer Biogasanlage zu einem Gasstromeingang der Abtrenneinrichtung und/oder zu einem Eduktgaseingang der Methanisierungseinrichtung.

Ein für den Einsatz in einer solchen Anlage einsetzbarer Methanisierungsreaktor kann als bauliche Einheit zusammen mit einem Elektrolyseur zur Herstellung wenigstens eines Teils des in dem Methanisierungsreaktor umgewandelten Wasserstoffes bereitgestellt werden.

Schließlich wird noch ein Gasgemisch mit sehr hohem Methangehalt unter Schutz gestellt, das nach einem Verfahren gemäß einem der obigen Verfahrensaspekte hergestellt (bereitgestellt) wurde.

Weitere Merkmale, Vorteile und Einzelheiten der Erfindung ergeben sich aus der folgenden Beschreibung mit Bezug auf die beigelegten Figuren, von denen
- Fig. 1: schematisch eine erste Ausführungsform der Erfindung zeigt,
- Fig. 2: schematisch den Aufbau einer am zweiten Aufbereitungsort aufgestellten Methanisierungsanlage zeigt,
- Fig. 3: eine weitere Ausführungsform der Erfindung zeigt, bei der ein unterschiedliches Basisgas für den Gasstrom verwendet wird

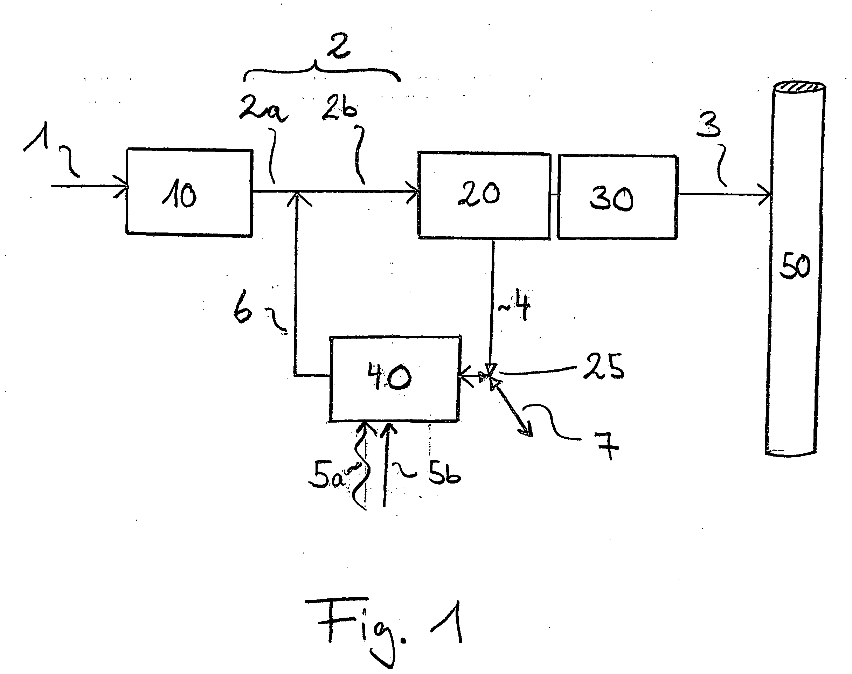

Anhand der schematischen Darstellung von Fig. 1 wird nun eine erste Ausführungsform der vorliegenden Erfindung beschrieben. Eine mit Biomasse 1 gespeiste Biogasanlage 10 erzeugt einen bereits entschwefelten Biorohgasstrom 2, welcher zu Zwecken der vereinfachten Darstellung (d.h. unter Vernachlässigung von Restbestandteilen) aus 60% Methan und 40% Kohlendioxid besteht. Tatsächlich enthält das Rohbiogas daneben noch unvermeidliche Verunreinigungen, insbesondere Wasserdampf, Stickstoff, Sauerstoff, Wasserstoff, Ammoniak sowie ggf. Restschwefelwasserstoff in sehr geringen Konzentrationen. Das pro Stunde vor Zuleitung eines Produktgases 6 strömende Rohbiogas 2a beträgt beispielsweise 1000 Normkubikmeter (1000 Nm³/h).

Rechnet man dieses Produktgas 6 nach dessen Einkopplung noch nicht hinzu, so stimmt der Rohgasstrom 2b mit dem Rohgasstrom 2a überein und erreicht eine Gasaufbereitungsanlage 20, in welcher durch ein geeignetes Trennverfahren, z.B. eine Gaswäsche oder ein Druckwechseladsorptionsverfahren (PSA), dem Rohbiogas das Kohlendioxid 4 entzogen wird. Vereinfacht gerechnet wird der Rohbiogasstrom von etwa 400 Nm³/h Gasstrommenge kohlendioxidbefreit und strömt entsprechend mit ca. 600 Nm³/h weiter. Bevor das so aufbereitete Rohbiogas, im folgenden Biomethan genannt, als einspeisefähiges Biomethan 3 in ein Gasnetz 50 eingespeist wird, wird es noch in einer Konditionierung 30 gemäß den für das Gasnetz 50 vorgesehenen Einspeisebedingungen konditioniert, wobei hier das Biomethan 3 als Austauschgas in beliebigen Mengen in das Gasnetz 50 eingespeist werden kann, da es einen Methangehalt von 99% oder mehr aufweist und in Bezug auf Trockenheit, Druck und Brennwert angepasst an das Gasnetz 50 eingespeist wird. Das Gasnetz 50 ist dabei auf die Speicherung und den Transport von H-Gas ausgelegt.

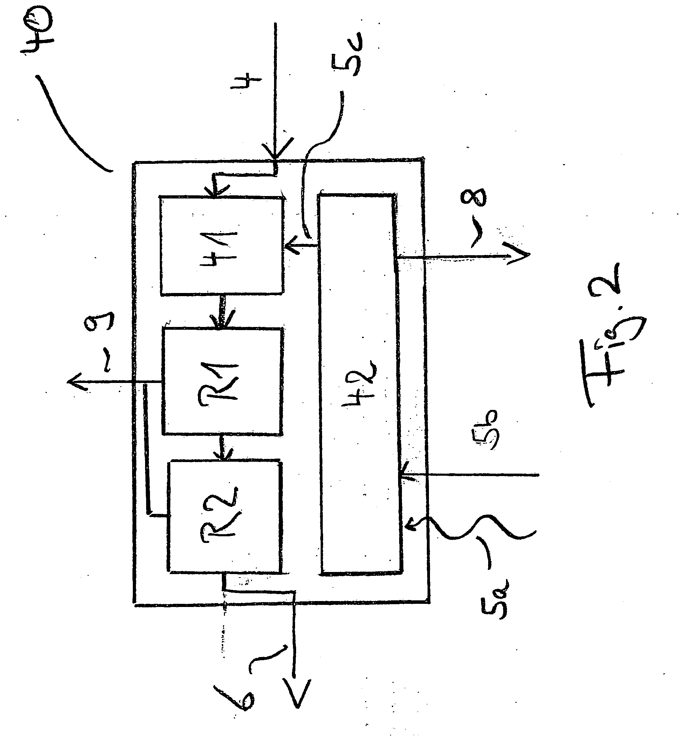

Das abgetrennte Kohlendioxid 4 wird nicht in die Umgebung freigesetzt, sondern nach z.B. seiner Rückgewinnung aus der Regenerierung der Gaswäscheflüssigkeit einer Methanisierungsanlage 40 zugeführt. In der in Fig. 2 genauer erläuterten Methanisierungsanlage 40 wird der zugeführte Kohlendioxidstrom zunächst mit Wasserstoff gemischt, welcher in der Anlage 40 elektrolytisch erzeugt wird, und anschließend in einer oder mehreren Reaktorstufen katalytisch methanisiert. Die Methanisierungsanlage 40 benötigt daher im wesentlichen nur eine Zuführung elektrischer Energie (Strom) 5a und eine Wasserzuführung 5b, während weitere Ausgangsprodukte wie Sauerstoff und Wärme geeignet verwendet werden können und hier nicht mehr weiter beschrieben werden.

Sofern die Methanisierungsanlage 40 auf Gasvolumenströme ausgelegt ist, die kleiner als der Gasvolumenstrom des Kohlendioxids 4 ist, beispielsweise auf einen Kohlendioxidvolumenstrom von 325 Nm³/h, kann am Ventil 25 überschüssiges Kohlendioxid 7 aus dem Kohlendioxidstrom 4 aus dem Leitungssystem ausgekoppelt werden und entweder freigesetzt oder anderweitig gespeichert oder verwendet werden.

Das aus der Methanisierungsanlage 40 strömende Produktgas 6, das in diesem Ausführungsbeispiel unter Vernachlässigung von Restverunreinigungen oder in sehr geringer Menge enthaltenen höheren Kohlenwasserstoffe aus ca. 92% Methan, 6% Kohlendioxid und 2% Wasserstoff besteht, wird nicht unmittelbar in das Gasnetz 50 eingespeist. Vielmehr wird der Produktgasstrom 6 in den Rohbiogasstrom 2 eingekoppelt und gelangt so ebenfalls in die Gasaufbereitungsanlage 20. Der in Betrieb der Methanisierungsanlage 40 eingekoppelte Produktgasstrom 6 sorgt somit dafür, dass eine Gesamtgasstrommenge von hier z.B. 1325 Nm³/h die Abtrennanlage 20 erreicht und nach Ausfilterung des Kohlendioxids in der Abtrennanlage 20 ein Biomethanstrom 3 von ca. 925 Nm³/h erzeugt wird und in dieser Volumenstrommenge konditioniert in das Gasnetz 50 eingespeist werden kann.

Durch die Aufbereitung des abgetrennten Kohlendioxids 4 in der Methanisierungsanlage 40 und die Rückführung des von der Methanisierungsanlage 40 erzeugten Produktgases 6 vor der Abtrenneinlage 20 wird somit die Menge des in das Gasnetz 50 eingespeisten Biomethans 3 mit sehr hohem Methangehalt von 99% oder mehr um 50% gegenüber derjenigen Situation gesteigert, in welche diese weitere Aufbereitung und Einkopplung nicht besteht. Desweiteren wird, auch wenn der Wasserstoff nicht zusätzlich abgetrennt wird (Beispiel Aminwäsche), aufgrund des Mischverhältnisses aus Rohbiogasstrom 2a und Produktgas 6 ein Wasserstoffgehalt des eingespeisten Endprodukts von weniger als 1% erreicht.

In Fig. 2 ist die Methanisierungsanlage 40 aus Fig. 1 nochmals detaillierter dargestellt. Sie ist als eine Aufbereitungseinheit ausgebildet, bei der der zugeführte Kohlendioxidstrom 4 in einen Mischraum 41 geleitet wird, in welchen ebenfalls Wasserstoff 5c geleitet wird, der in einem Elektrolyseur 42 erzeugt wird. Der Elektrolyseur 42 hat einen Wassereingang für das elektrolytisch zu trennende Wasser 5b und einen Stromeingang- zur Stromzuführung 5a sowie einen Ausgang für das Beiprodukt der Elektrolyse, Sauerstoff 8.

Nach dem Mischen des Kohlendioxids 4 und des Wasserstoffs 5c im Mischraum 41 in einem Verhältnis, das im wesentlichen durch das stöchiometrische Verhältnis der Kohlendioxidmethanisierungsreaktion festgelegt ist, wird das Wasserstoff-Kohlendioxidgasgemisch in einem aus zwei Reaktorstufen R1 und R2 bestehenden Methanisierungsreaktor katalytisch methanisiert und dabei das Produktgas 6 mit bereits hohem Methangehalt von z.B. 92% erzeugt. Die bei dem exothermen Methanisierungsprozess gewonnene Wärme 9 kann ebenfalls aus der Aufbereitungseinheit 40 entnommen und wie auch der Sauerstoff 8 geeignet weitergenutzt werden. Es kann jedoch auch ein Reaktor mit nur einer, drei oder noch mehr Reaktorstufen verwendet werden.

Einzelheiten von möglichen Steuerungen einer solchen mehrstufigen katalytischen Methanisierung sind beispielsweise in der noch nicht veröffentlichten Anmeldung PCT/EP2010/006877 beschrieben und werden hier nicht weiter erläutert.

Sofern die Trenneinrichtung 20 nicht vorsieht, dass das über den Produktgasstrom 6 in den Rohbiogasstrom 2a eingekoppelte, in dem Produktgas 6 enthaltenden Restwasserstoffanteil ebenfalls abtrennt, ist dieser Wasserstoffgehalt in entsprechend dem Mischverhältnis aus ausgehendem Rohbiogasstrom 2a und eingekoppeltem Produktgas 6 verdünnten Verhältnis im Biomethanstrom 3 noch enthalten. In diesem Fall wird die Aufbereitungsanlage 40 durch gegenüber dem exakten stöchiometrischen Verhältnis zur Methanherstellung höherer Kohlendioxidzuführung 4 bzw. niedrigerer Wasserstoffzuführung 5c derart gesteuert, dass der Wasserstoffanteil im Biomethanstrom 3 nur noch 1% oder weniger beträgt. Dies bedeutet für die anhand des Beispiels von Fig. 1 erläuterten Gasvolumenströme einen Wasserstoffgehalt im Produktgas 6 von ca. 4% oder weniger.

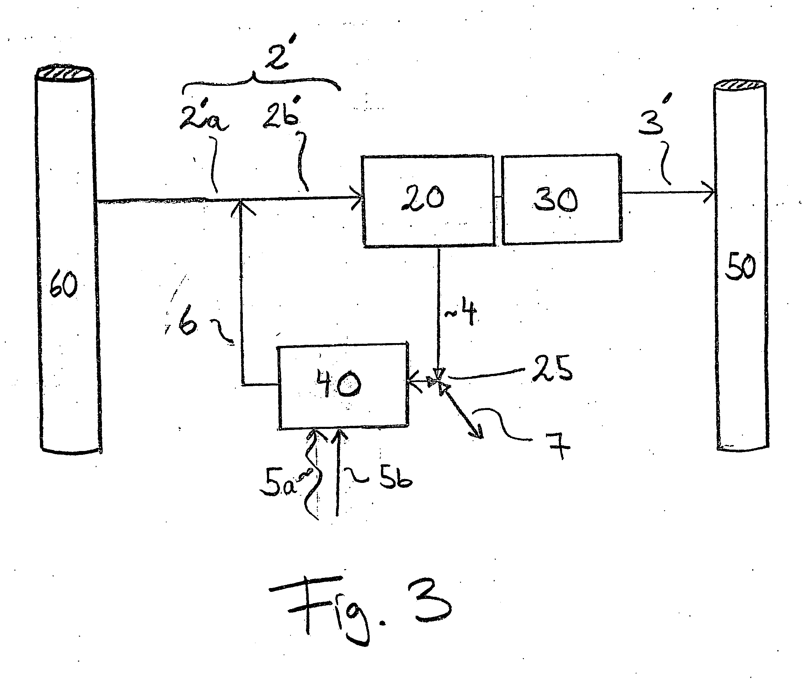

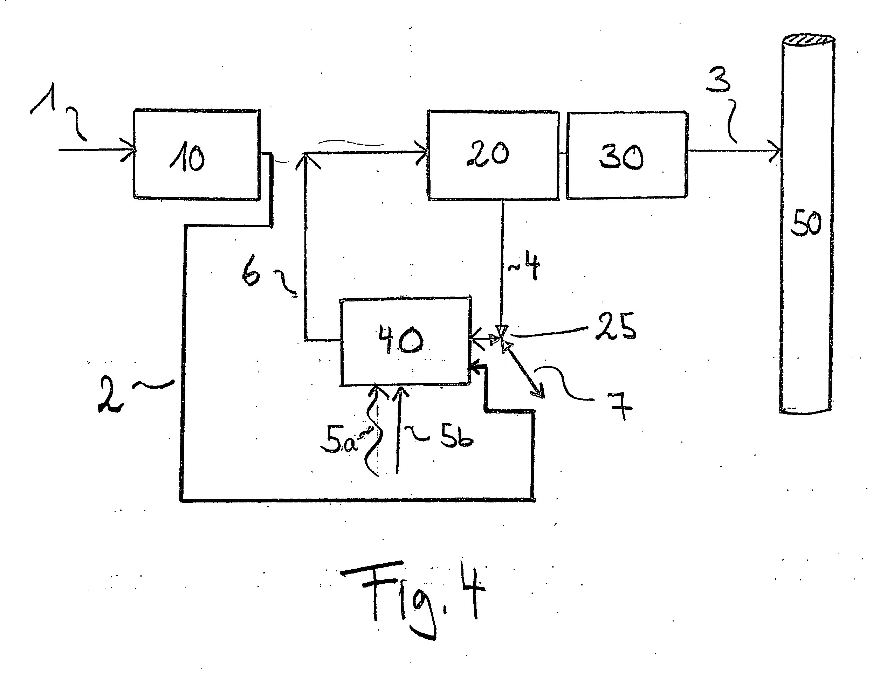

In Fig. 3 ist eine weitere Ausführungsform der Erfindung schematisch dargestellt. Gegenüber dem Ausführungsbeispiel von Fig. 1 ist der dortige Ausgangs-Biorohgasstrom 2a nunmehr durch einen Basisgasstrom bzw. ein Basisgas 2'a ersetzt, welches von einer unterschiedlichen Quelle stammt, nämlich einem weiteren Gasnetz 60. Die Einkopplung der Methanisierungsanlage 40 mit deren Produktgasstrom 6 stromaufwärts der Trenneinrichtung 20 ist genauso gestaltet wie in dem Ausführungsbeispiel von Fig. 1, und zur Vermeidung von Wiederholungen wird auf eine erneute Erläuterung derselben verzichtet. Anstelle des Biomethans 3 wird somit ein Gasgemisch 3' mit sehr hohem Methangehalt von 99% oder mehr in das Gasnetz 50 eingespeist.

Bei dem Gasnetz 60 kann es sich z.B. um ein Gasnetz handeln, das Gas in L-Gasqualität speichert und transportiert. Auf diese Weise kann das in Fig. 3 abgebildete Gesamtsystem somit L-Gas in Erdgassubstitute in H-Gasqualität veredelt werden, ohne dass dabei Kohlendioxid aus der Reaktionskette ausgekoppelt werden muss. Auch hier kann der für die Veredelung benötigte Bedarf an elektrischer Energie aus erneuerbaren Energien bereitgestellt werden, bevorzugt wenn diese große Mengen Energie in das Stromnetz einspeisen, wodurch das Stromnetz ebenfalls entlastet wird. In alternativen Ausführungsformen könnte das Gasnetz 60 auch der Erdkruste entnommenes Rohgas, Gas aus Syntheseprozessen, Gas aus jeder Art der Vergärung - z.B. einer Mülldeponie - repräsentieren.

In einer Abwandlung dieser Ausführungsform kann das Gasnetz 50 auch mit dem Gasnetz 60 übereinstimmen. Das System um die Methanisierungsanlage 40 dient dann der Erhöhung des Methangehalts in dem Gasnetz insbesondere unter Vermeidung von CO₂-Freigabe.

Die anhand der Figuren erläuterten Ausführungsbeispiele der Erfindung sind nicht als Einschränkung der Erfindung auszulegen. Vielmehr können einzelne Merkmale der Beschreibung wie auch der Ansprüche für die Ausführung der Erfindung in ihren verschiedenen Ausführungsformen wesentlich sein.

## Patentansprüche

1. Verfahren zur Bereitstellung eines Gases mit sehr hohem Methangehalt, bei dem man den Methangehalt eines sowohl Kohlendioxid als auch Methan enthaltenden Gasstroms durch eine an einem ersten Aufbereitungsort erfolgende Abtrennung wenigstens eines Teils des in dem Gasstrom enthaltenen Kohlendioxids auf wenigstens 96 Vol.% erhöht,
wobei ein an einem zweiten Aufbereitungsort durch katalytische Methanisierung eines sowohl Kohlendioxid als auch Wasserstoff enthaltenden Eduktgases erzeugtes Produktgas wenigstens einen Teil des Gasstromes vor dessen Erreichen des ersten Aufbereitungsortes bildet, und wobei man zur Bildung des Gasstromes das Produktgas einem bereits Kohlendioxid und Methan enthaltenden Basisgas zuführt,
**dadurch gekennzeichnet, dass** man das aus dem Gasstrom abgetrennte Kohlendioxid dem zweiten Aufbereitungsort zuleitet, dieses zugeleitete Kohlendioxid wenigstens ein Teil des Kohlendioxids des Eduktgases bildet, und dass wenigstens ein Großteil des Wasserstoffes des Eduktgases elektrolytisch erzeugt wird.

2. Verfahren nach Anspruch 1, bei dem das zugeleitete Kohlendioxid den gesamten Kohlendioxidanteil des Eduktgases bildet.

3. Verfahren nach Anspruch 1 oder 2, bei dem man auch Wasserstoff wenigstens teilweise aus dem Gasstrom abtrennt und insbesondere dem Eduktgas zuführt.

4. Verfahren nach einem der Ansprüche 1 bis 3, bei dem das Basisgas ein aus einer Biogasanlage gewonnenes Gas, insbesondere ein bereits entschwefeltes Rohbiogas ist, das insbesondere bereits aufgrund seines Entstehungsprozesses nur einen minimalen Gehalt an Luftstickstoff und Sauerstoff aufweist.

5. Verfahren nach einem der Ansprüche 1 bis 3, bei dem das Basisgas einem unter vorgegebenen Einspeisebedingungen stehenden Gasnetz entnommen ist, insbesondere einem L-Gasnetz.

6. Verfahren nach einem der Ansprüche 1 bis 3, bei dem das Eduktgas wenigstens zum Teil, insbesondere vollständig aus einem bereits Kohlendioxid und Methangas enthaltenden Basisgas, insbesondere Rohbiogas besteht, welchem Wasserstoff zugegeben wurde.

7. Verfahren nach einem der Ansprüche 1 bis 6, bei dem man das bereitgestellte Gas mit dem sehr hohen Methangehalt gemäß vorgegebenen Einspeisebedingungen für eine Einspeisung in ein den Einspeisebedingungen unterliegendes Gasnetz konditioniert und insbesondere daraufhin in das Gasnetz einspeist.

8. Verfahren nach einem der Ansprüche 1 bis 7, bei dem die elektrolytische Wasserstofferzeugung nahe dem zweiten Aufbereitungsort erfolgt.

9. Verfahren nach Anspruch 8, bei dem eine Zugabe des kohlendioxidhaltigen Teils des Eduktgases in Abhängigkeit der für die Elektrolyse insbesondere zeitlich schwankend zur Verfügung gestellten elektrischen Energie gesteuert wird.

10. Verfahren nach einem der vorhergehenden Ansprüche, bei dem man die Methanisierung auf eine Verschiebung der Wasserstoffkonzentration des Produktgases hin zu geringen Wasserstoffgehalten steuert, insbesondere auf Kosten eines höheren Kohlen-dioxidgehaltes.

11. Verfahren nach einem der vorhergehenden Ansprüche, bei dem zum Abtrennen des Kohlendioxidanteils aus dem Gasstrom und/oder zum Abtrennen des Wasserstoffes daraus eine Gaswäsche, Aminwäsche, und/oder ein Druckwechseladsorptionsverfahren (PSA) zum Einsatz kommt.

12. Anlage zur Bereitstellung eines Gases mit sehr hohem Methangehalt von wenigstens 96 Vol.%, die zur Durchführung eines Verfahrens gemäß einem der vorhergehenden Ansprüche ausgelegt ist, mit
einer Einrichtung (20) zur Erhöhung des Methangehalts eines ihr zugeführten und sowohl Kohlendioxid als auch Methan enthaltenden Gasstroms (2b) durch Abtrennen wenigstens eines Teils (4) des Kohlendioxids daraus,
einer einen Methanisierungsreaktor (40) aufweisenden Einrichtung (40; R1, R2) zur Herstellung eines Produktgases (6) durch katalytische Methanisierung eines sowohl Kohlendioxid als auch Wasserstoff enthaltenden Eduktgases,
einem Leitungssystem, das stromaufwärts der Abtrenneinrichtung durch eine Zuführung des Produktgases zu einem bereits Kohlendioxid und Methan enthaltenden Basisgas eine teilweise Bildung des Gasstroms (1b) durch das Produktgas der Methanisierungseinrichtung und das durch eine Verbindung des Ausgangs der Abtrenneinrichtung für das abgetrennte Kohlendioxid zu einem Gaseingang der Methanisierungseinrichtung eine wenigstens teilweise Bildung des Eduktgases aus dem abgetrennten Kohlendioxid erlaubt,
einem Elektrolyseur (42) zur Herstellung wenigstens eines Teils (5c) des in dem Methanisierungsreaktor umgewandelten Wasserstoffes, und
einer die Anlage gemäß einer Verfahrensgestaltung nach einem der vorhergehenden Ansprüche steuernden Steuereinrichtung.

13. Anlage nach Anspruch 12, wobei das Leitungssystem eine Verbindung des Ausgangs der Abtrenneinrichtung (20) für abgetrennten Wasserstoff zu einem Gaseingang der Methanisierungseinrichtung, sowie insbesondere eine Verbindung eines Gasausgangs einer Biogasanlage zu einem Gasstromeingang der Abtrenneinrichtung (20) aufweist.

14. Anlage nach einem der Ansprüche 12 oder 13, bei der der Methanisierungsreaktor (40) als Einheit zusammen mit dem Elektrolyseur (42) gestaltet ist.

## Claims

1. A method for providing a gas with very high methane content wherein the methane content of a gas stream containing both carbon dioxide and methane is increased to at least 96 vol% by separating at least a portion of the carbon dioxide contained in the gas stream at a first treatment site,
wherein a product gas produced at a second treatment site by catalytic methanization of a reactant gas containing both carbon dioxide and hydrogen forms at least a part of the gas stream before reaching the first treatment site, and wherein the product gas is supplied to a base gas already containing carbon dioxide and methane to form the gas stream,
**characterised in that** the carbon dioxide separated from the gas stream is fed to the second treatment site, this fed carbon dioxide forming at least a part of the carbon dioxide of the reactant gas, and **in that** at least a major part of the hydrogen of the reactant gas is produced electrolytically.

2. The method according to claim 1, wherein the fed carbon dioxide forms the total carbon dioxide content of the reactant gas.

3. The method according to claim 1 or 2, wherein hydrogen is also at least partially separated from the gas stream and in particular supplied to the reactant gas.

4. The method according to one of claims 1 to 3, wherein the base gas is a gas obtained from a biogas plant, in particular an already desulphurised raw biogas, which has only a minimal content of atmospheric nitrogen and oxygen, in particular owing to its formation process.

5. The method according to one of claims 1 to 3, wherein the base gas is taken from a gas network under predetermined feed-in conditions, in particular a low calorific value gas network.

6. The method according to one of claims 1 to 3, wherein the reactant gas consists at least in part, in particular entirely, of a base gas already containing carbon dioxide and methane gas, in particular raw biogas, and added hydrogen.

7. The method according to one of claims 1 to 6, wherein the provided gas with very high methane content is conditioned in accordance with predetermined feed-in conditions for feeding into a gas network subject to the feed-in conditions and, in particular, subsequently fed into the gas network.

8. The method according to one of claims I to 7, wherein the electrolytic hydrogen production takes place near the second treatment site.

9. The method according to claim 8, wherein an addition of the carbon-dioxide-containing portion of the reactant gas is controlled as a function of the electrical energy which is provided for electrolysis and which, in particular, varies over time.

10. The method according to one of the preceding claims, wherein methanation is controlled to shift the hydrogen concentration of the product gas towards low hydrogen contents, in particular at the expense of a higher carbon dioxide content.

11. The method according to one of the preceding claims, wherein a gas wash, amine wash and/or a pressure swing adsorption process (PSA) is used to separate the carbon dioxide fraction from the gas stream and/or to separate the hydrogen therefrom.

12. A plant for providing a gas with a very high methane content of at least 96 vol%, adapted to carry out a method according to any of the preceding claims, comprising
a device (20) for increasing the methane content of a gas stream (2b) supplied to said device and containing both carbon dioxide and methane by separating at least a portion (4) of the carbon dioxide therefrom,
a device (40; R1, R2) comprising a methanation reactor (40) for producing a product gas (6) by catalytic methanation of an reactant gas containing both carbon dioxide and hydrogen,
a conduit system which, upstream of the separation device, by supplying the product gas to a base gas already containing carbon dioxide and methane, permits partial formation of the gas flow (1b) by the product gas of the methanation device and which, by connecting the outlet of the separation device for the separated carbon dioxide to a gas inlet of the methanation device, permits at least partial formation of the reactant gas from the separated carbon dioxide,
an electrolyzer (42) for producing at least a portion (5c) of the hydrogen converted in the methanation reactor, and
a control device controlling the plant according to a process design according to one of the previous claims.

13. The plant according to claim 12, wherein the conduit system comprises a connection of the outlet of the separation device (20) for separated hydrogen to a gas inlet of the methanation device, and in particular a connection of a gas outlet of a biogas plant to a gas flow inlet of the separation device (20).

14. The plant according to one of claims 12 and 13, wherein the methanation reactor (40) is designed as a unit together with the electrolyser (42).

## Revendications

1. Procédé de production d'un gaz à très haute teneur en méthane, dans lequel on élève à au moins 96 % en volume la teneur en méthane d'un flux gazeux contenant du dioxyde de carbone ainsi que du méthane en effectuant, dans un premier lieu d'élaboration, une séparation d'au moins une partie du dioxyde de carbone contenu dans le flux gazeux,
dans lequel un produit gazeux produit dans un deuxième lieu d'élaboration par méthanisation catalytique d'un réactif gazeux contenant du dioxyde de carbone ainsi que de l'hydrogène constitue au moins une partie du flux gazeux avant son arrivée dans le premier lieu d'élaboration, et dans lequel on apporte le produit gazeux à un gaz de base contenant déjà du dioxyde de carbone et du méthane en vue de former le flux gazeux,
**caractérisé en ce que** l'on transfère le dioxyde de carbone séparé du flux gazeux auprès du deuxième lieu d'élaboration, ce dioxyde de carbone transféré constituant au moins une partie du dioxyde de carbone du réactif gazeux, et **en ce qu'**au moins une majeure partie de l'hydrogène du réactif gazeux est produit par voie électrolytique.

2. Procédé selon la revendication 1, dans lequel le dioxyde de carbone transféré constitue la totalité de la part de dioxyde de carbone du réactif gazeux.

3. Procédé selon la revendication 1 ou 2, dans lequel on sépare également au moins partiellement l'hydrogène du flux gazeux et on l'apporte notamment au réactif gazeux.

4. Procédé selon l'une des revendications 1 à 3, dans lequel le gaz de base est un gaz produit dans une installation de biogaz, notamment un biogaz brut préalablement désulfuré, lequel présente notamment, en raison même de son processus d'élaboration, une teneur minimale en azote et en oxygène.

5. Procédé selon l'une des revendications 1 à 3, dans lequel le gaz de base provient d'un réseau de gaz maintenu dans des conditions d'injection prédéfinies, notamment un réseau de gaz à faible pouvoir calorifique.

6. Procédé selon l'une des revendications 1 à 3, dans lequel le réactif gazeux consiste au moins en partie, notamment totalement, en un gaz de base contenant déjà du dioxyde de carbone et du gaz méthane, notamment du biogaz brut, auquel a été ajouté de l'hydrogène.

7. Procédé selon l'une des revendications 1 à 6, dans lequel le gaz à très haute teneur en méthane produit est conditionné selon des conditions d'injection prédéfinies pour une injection dans un réseau de gaz soumis aux conditions d'injection, après quoi il est notamment injecté dans le réseau de gaz.

8. Procédé selon l'une des revendications 1 à 7, dans lequel la production électrolytique d'hydrogène s'effectue à proximité du deuxième lieu d'élaboration.

9. Procédé selon la revendication 8, dans lequel l'ajout de la partie du réactif gazeux contenant du dioxyde de carbone est régulé en fonction de l'énergie électrique mise à disposition de manière notamment variable dans le temps pour l'électrolyse.

10. Procédé selon l'une quelconque des revendications précédentes, dans lequel on régule la méthanisation pour un passage de la concentration en hydrogène du produit gazeux vers de moindres teneurs en hydrogène, notamment au prix d'une plus forte teneur en dioxyde de carbone.

11. Procédé selon l'une quelconque des revendications précédentes, dans lequel un lavage du gaz, un lavage aux amines et/ou un procédé d'adsorption par inversion de pression (PSA) sont mis en oeuvre pour séparer la part de dioxyde de carbone et/ou de l'hydrogène du flux gazeux.

12. Installation de production d'un gaz à très haute teneur en méthane, de l'ordre d'au moins 96 % en volume, conçue pour mettre en oeuvre un procédé selon l'une des revendications précédentes, comprenant
un dispositif (20) d'élévation de la teneur en méthane d'un flux gazeux (2b), contenant du dioxyde de carbone ainsi que du méthane, qui lui est apporté, par séparation d'au moins une partie (4) du dioxyde de carbone,
un dispositif (40 ; R1, R2) présentant un réacteur de méthanisation (40) pour produire un produit gazeux (6) par méthanisation catalytique d'un réactif gazeux contenant du dioxyde de carbone ainsi que de l'hydrogène,
un système de conduites qui permet, en amont du dispositif de séparation, grâce à l'apport du produit gazeux à un gaz de base contenant déjà du dioxyde de carbone et du méthane, une formation partielle du flux gazeux (1b) par le biais du produit gazeux du dispositif de méthanisation, et qui permet, en reliant la sortie du dispositif de séparation, destinée au dioxyde de carbone séparé, à une entrée de gaz du dispositif de méthanisation, une formation au moins partielle du réactif gazeux à partir du dioxyde de carbone séparé,
un électrolyseur (42) destiné à produire au moins une partie (5c) de l'hydrogène transformé dans le réacteur de méthanisation, et
un dispositif de commande qui commande l'installation suivant une procédure selon l'une quelconque des revendications précédentes.

13. Installation selon la revendication 12, dans laquelle le système de conduites présente une liaison de la sortie du dispositif de séparation (20), destinée à l'hydrogène séparé, à une entrée de gaz du dispositif de méthanisation, ainsi notamment qu'une liaison d'une sortie de gaz d'une installation de biogaz à une entrée pour flux gazeux du dispositif de séparation (20).

14. Installation selon l'une des revendications 12 et 13, dans laquelle le réacteur de méthanisation (40) est conçu de façon unitaire avec l'électrolyseur (42).
